# EUROPEAN PATENT SPECIFICATION

(11) **EP 3 858 301 B1**
(45) Date of publication and mention of the grant of the patent: **28.10.2026**
(21) Application number: 20192726.6
(22) Date of filing: 06.03.2014
(51) Int. Cl.: A61F 2/966, A61F 2/07, A61F 2/962, A61F 2/97

(54) **ENDOPROSTHESIS DELIVERY SYSTEMS WITH DEPLOYMENT AIDS**
SYSTEME ZUR FREISETZUNG VON ENDOPROTHESEN MIT FREISETZUNGSHILFE
SYSTÈMES DE POSE D'ENDOPROTHÈSE AYANT DES AIDES DE DÉPLOIEMENT

(30) Priority: 14.03.2013 US 201361782134 P; 05.03.2014 US 201414198037
(43) Date of publication of application: 04.08.2021
(60) Divisional application: 26205649.2
(62) Document number(s) of the earlier application(s) in accordance with Art. 76 EPC: 14716466.9 / 2 967 953
(73) Proprietor: W. L. Gore & Associates, Inc., Newark, DE 19711 (US)
(72) Inventor: ARMSTRONG, Joseph, R., Newark, DE 19711 (US); CULLY, Edward, H., Newark, DE 19711 (US); DUNCAN, Jeffrey, B, Newark, DE 19711 (US); KOVACH, Larry J., Newark, DE 19711 (US); PAJOT, Douglas, F., Newark, DE 19711 (US); SHORT, Brandon, C., Newark, DE 19711 (US); ULM, Mark, J., Newark, DE 19711 (US); VONESH, Michael, J., Newark, DE 19711 (US)
(74) Representative: HGF

(56) References cited:
- US-A1- 2011 301 702
- US-B2- 7 771 463

## Description

### FIELD

The present disclosure generally relates to endoprostheses for treating diseases of the vasculature and similar anatomies, and more particularly to endoprosthesis delivery systems for preventing entanglement of covering members with such endoprostheses.

### BACKGROUND

Many endoprostheses (or endoprosthetic devices), such as, for example, stent-grafts, are constructed to reinforce, replace, bridge, or otherwise treat a part of a blood vessel. An endoprosthesis may thus guide blood flow through a lumen defined by a generally tubular interior of such a vessel. Other tubular endoprostheses are designed for use in other body regions, for example, the esophagus, ureters, gastrointestinal tract and various ducts.

In many cases, endoprostheses are constrained within a covering member or sheath, and when the covering member is removed, as during deployment, the devices expand, are expanded under force or self-expand to assume a larger diameter. US Patent No. 7,771,463 discloses medical devices and methods for delivery or implantation of tubular prostheses within hollow body organs and vessels or other luminal anatomy. A prosthesis is held in a compressed form upon an inner wire of a delivery guide by twisting the device. From time to time, however, as a covering member is removed, the member may snag, catch, or become entangled on the endoprosthesis. Thus, improved endoprosthesis delivery systems are desirable.

### SUMMARY

The invention is defined in appended independent claim 1, further embodiments are described in the dependent claims. Methods of surgery described here below are not claimed but are useful for the understanding of the invention.

The present disclosure includes an endoprosthesis delivery system comprising an elongate member, such as a catheter, an endoprosthesis, a covering member disposed about the endoprosthesis, and at least one flexible element situated between the endoprosthesis and the covering member. The covering member can extend beyond an end of the endoprosthesis. In operation, as the covering member is removed, the flexible element can guide the covering member over the end of the endoprosthesis to prevent entanglement between the end of the endoprosthesis and the covering member.

The present disclosure further includes an endoprosthesis delivery system comprising an elongate member having a slip resistant section or material, an endoprosthesis, and a covering member. The slip resistant material can comprise a gummy or mildly sticky material or a compressible material as described, and it can extend beyond a distal and/or proximal end of the endoprosthesis. In operation, as the covering member unravels or is otherwise removed to release the endoprosthesis, the slip resistant material can impede bunching of the cover. This can, in turn, prevent, or help to prevent, entanglement between the covering member and the endoprosthesis.

The present disclosure further includes an endoprosthesis delivery system comprising an elongate member, an endoprosthesis having a distal and/or proximal edge, an end cap adjacent to one of these edges, and a covering member. The covering member can extend beyond an end of the endoprosthesis and over a surface of the end cap. In operation, as the covering member is removed, the end cap can prevent the covering member from becoming entangled with an edge of the endoprosthesis. Rather, the covering member can follow the end cap as a guide over the edges of the endoprosthesis.

### BRIEF DESCRIPTION OF THE DRAWINGS

The features and advantages of the present disclosure will become more apparent from the detailed description set forth below when taken in conjunction with the drawings, wherein:
Figure 1 illustrates a perspective view of an endoprosthesis delivery system;
Figure 2 illustrates a magnified perspective view of an endoprosthesis delivery system;
Figure 3 illustrates a perspective view of an endoprosthesis delivery system, including a line along which a cross-sectional view is to be taken;
Figure 4 illustrates a cross-sectional view of an endoprosthesis delivery system taken along the line shown in Figure 3;
Figure 5 illustrates a perspective view of an endoprosthesis delivery system without a compressible material;
Figure 6 illustrates a perspective view of an endoprosthesis delivery system with a compressible material;
Figure 7 illustrates a perspective view of an endoprosthesis delivery system, including a line along which a cross-sectional view is to be taken;
Figure 8 illustrates a cross-sectional view of an endoprosthesis delivery system taken along the line shown in Figure 7;
Figure 9 illustrates a magnified and cropped cross-sectional view of an endoprosthesis delivery system taken along the line shown in Figure 7;
Figure 10 illustrates a perspective view of an endoprosthesis delivery system in the process of deploying an endoprosthesis;
Figure 11 illustrates a magnified perspective view of an endoprosthesis delivery system in the process of deploying an endoprosthesis;
Figure 12A illustrates perspective view of an endoprosthesis delivery system including a slip resistant material;
Figure 12B illustrates a perspective view of an endoprosthesis delivery system including a flexible, longitudinally compressed polymeric member;
Figure 13A illustrates a perspective view of a method of manufacturing an endoprosthesis delivery system comprising rolling a sheet of compliant material about a mandrel;
Figure 13B illustrates a perspective view of a method of manufacturing an endoprosthesis delivery system comprising curing a rolled sheet of compliant material;
Figure 13C illustrates a perspective view of a method of manufacturing an endoprosthesis delivery system comprising longitudinally compressing a rolled sheet of compliant material to form a slip resistant material;
Figure 14 illustrates a perspective view an endoprosthesis delivery system having an end cap and a tapered end cap;
Figure 15 illustrates a perspective view of an endoprosthesis delivery system in a post-deployment stage;
Figure 16 illustrates a cross-sectional view of a tapered end cap;
Figure 17 illustrates a process for loading an endoprosthesis on an inner shaft of an elongate member and within a covering member;
Figure 18a illustrates a perspective view of an endoprosthesis delivery system prior to constraining an endoprosthesis in which a flexible element is fully opened;
Figure 18b illustrates a shape of a flexible element when viewed axially;
Figure 18c illustrates a constrained endoprosthesis with a flexible element placed between an endoprosthesis and an endoprosthesis covering element;
Figure 19 illustrates a cross-sectional view of an endoprosthesis delivery system;
Figure 20A illustrates a film tube capable of forming a portion of an endoprosthesis delivery system;
Figure 20B illustrates a film tube including a plurality of slits for forming a portion of an endoprosthesis delivery system; and
Figure 21 illustrates an endoprosthesis delivery system.

### DETAILED DESCRIPTION OF THE ILLUSTRATED EMBODIMENTS

Persons skilled in the art will readily appreciate that various aspects of the present disclosure can be realized by any number of methods and apparatuses configured to perform the intended functions. Stated differently, other methods and apparatuses can be incorporated herein to perform the intended functions. It should also be noted that the accompanying drawing figures referred to herein are not all drawn to scale, but may be exaggerated to illustrate various aspects of the present disclosure, and in that regard, the drawing figures should not be construed as limiting. Finally, although the present disclosure may be described in connection with various principles and beliefs, the present disclosure should not be bound by theory.

The terms "endoprosthetic device," "endoprosthesis," "vascular device," and the like can refer, throughout the specification and in the claims, to any medical device capable of being implanted and/or deployed within a body lumen. In various embodiments, an endoprosthesis can comprise a stent, a stent-graft, graft, a filter, an occluder, a balloon, a lead, and energy transmission device, a deployable patch, an indwelling catheter, and the like.

In addition, throughout this specification and claims, the delivery systems described herein can, in general, include an endoprosthesis constrained by a "covering member" or "sheath." The covering member or sheath can, in various embodiments, comprise a sheet of material that is fitted about an endoprosthesis. The covering member or sheath can, in various embodiments, comprise a plurality of knitted fibers located about the endoprosthesis. These fibers can, for example, comprise a woven warp knit or knit-braid, as described in United States Patent No. 6,315,792 to Armstrong et al., issued November 13, 2001, entitled "Remotely removable covering and support,". The covering member can be coupled to a pull line extending down the length of the catheter, which a physician can pull to facilitate uncovering the endoprosthesis.

For example, a covering member comprising a plurality of fibers can be coupled to a pull line, which a physician can pull to unravel the plurality of fibers. Thus, the covering member can be characterized as "unzipping", in that the pull line causes the covering member to open or unzip along a straight line. In addition, in various embodiments, a covering member can be unzipped, first, along a proximal vector and, second, along a distal vector. In various embodiments, a covering member can be unzipped along a longitudinal vector running substantially parallel to the longitudinal axis of an elongate member.

Covering members can become caught on the endoprosthesis during deployment. For example, the covering member comprising a plurality of fibers can extend beyond an end or edge of an endoprosthesis, and as the covering member begins to be removed, e.g., unzipped, along a particular vector (e.g., a longitudinal vector), the member can become entangled with the end of the endoprosthesis. For example, the covering member can become entangled with one or more apices comprising the end of the endoprosthesis. Although the following description may often refer to a covering member comprising a plurality of fibers; in various embodiments, a covering member can comprise any of a variety of covers or sheaths capable of constraining an endoprosthesis.

As used throughout the specification and in the claims, the term "elongate member" can refer to a shaft-like structure such as a catheter, guidewire, introducer sheath, or the like. In various embodiments, an endoprosthesis can be mounted or loaded on a catheter, also referred to herein as an inner shaft, and, in a constrained diameter, fit within an introducer sheath, also referred to herein as an outer shaft.

Further, throughout this specification and in the claims, the term "distal" refers to a relative location that is farther from a location in the body at which the medical device was introduced. Similarly, the term "distally" refers to a direction away from a location in the body at which the medical device was introduced.

The term "proximal" refers, throughout the specification and in the claims, to a relative location that is closer to the location in the body at which the medical device was introduced. Similarly, the term "proximally" refers to a direction towards a location in the body at which the medical device was introduced.

With continuing regard to the terms proximal and distal, this disclosure should not be narrowly construed with respect to these terms. Rather, the devices and methods described herein may be altered and/or adjusted relative to the anatomy of a patient.

As used herein, the term "constrain" may mean (i) to limit expansion, occurring either through self-expansion or expansion assisted by a device, of the diameter of an expandable implant, or (ii) to cover or surround, but not otherwise restrain, an expandable implant (e.g., for storage or biocompatibility reasons and/or to provide protection to the expandable implant and/or the vasculature).

Accordingly, while specific embodiments are described in greater detail below, in general, the present disclosure will focus primarily upon endoprosthesis delivery systems, and in particular, systems for delivery of endoprostheses covered by a covering member combined with mechanisms that prevent snagging or entanglement between the covering member and the endoprosthesis. Methods for the delivery of endoprosthesis delivery systems disclosed herein do not fall within the scope of the invention.

In various embodiments, an endoprosthesis delivery system can comprise an elongate member, such as a catheter, an endoprosthesis, a covering member disposed about the endoprosthesis, and at least one flexible element situated between the endoprosthesis and the covering member. The covering member can extend beyond an end of the endoprosthesis. The endoprosthesis delivery system can further comprise a compressible material section of the elongate member extending at least beneath an end of the stent.

In operation, as the covering member unravels, the flexible element can guide the covering member over the end of the endoprosthesis to prevent a catch, snag, or entanglement between the end of the endoprosthesis and the covering member. For example, in an embodiment where the covering element comprises a plurality of fibers and is removed in a proximal direction, the flexible element can overlay a distal edge of the endoprosthesis, and as the covering member unravels, one or more fibers can ride over the flexible element. Similarly, in another embodiment where the covering member is removed in a distal direction, the flexible element can guide one or more fibers over a proximal edge of the endoprosthesis. Thus, the flexible element can prevent or reduce the likelihood of the covering member becoming caught on the endoprosthesis during deployment of the device. Optionally, the flexible element can extend through an opening at least partially defined by an edge, such as an apex, or an aperture formed in a graft member.

In the same or different embodiments, an endoprosthesis delivery system can comprise an elongate member, such as a catheter, having a slip resistant section or material, an endoprosthesis, and a covering member or sheath. The slip resistant material can comprise a gummy or sticky material, a compressible material, or a longitudinally compressed material (or various combinations thereof) that is located on a section of catheter adjacent a distal and/or proximal end of the endoprosthesis. The sheath, as above, can comprise a knitted plurality of fibers and can extend beyond the distal and/or proximal end of the endoprosthesis and be collocated with the slip resistant section.

During deployment, as the sheath unravels to release the endoprosthesis, the slip resistant material can impede bunching of the sheath fibers. This can, in turn, prevent, or help to prevent, a snag or entanglement between the sheath and the endoprosthesis. For example, the portion of the slip resistant material that extends beyond the distal and/or proximal end of the endoprosthesis can act as a mild adhesive between the sheath and the catheter, so that as the pull line coupled to the sheath is retracted (e.g., to initiate deployment), the sheath does not merely follow the motion of the pull line and slide down an otherwise relatively smooth surface of the catheter, bunching as it slides, to eventually become hung or entangled with an end of the endoprosthesis. Rather, the slip resistant material can releasably or mildly bind the sheath to the catheter, so that pulling of the pull line does not result in bunching that could lead to a failed deployment. In various embodiments wherein the sheath comprises a plurality of fibers, the fibers can partially embed into the slip resistant material as the pull line is pulled.

In different or the same embodiments, an endoprosthesis delivery system can comprise an elongate member, for example a catheter, an endoprosthesis having a distal and/or proximal edge, an end cap adjacent to one of these edges, and a covering member, such as a knitted covering member as described above. The covering member can extend beyond an end of the endoprosthesis and over a surface of the end cap.

By way of example, in operation, as the covering member comprising a plurality of fibers unravels to uncover or release the endoprosthesis from a delivery and/or constrained diameter, the end cap can prevent the fibers from becoming hung or entangled with an edge of the endoprosthesis. Rather, the covering member can follow the end cap surface guiding it over the edges of the endoprosthesis.

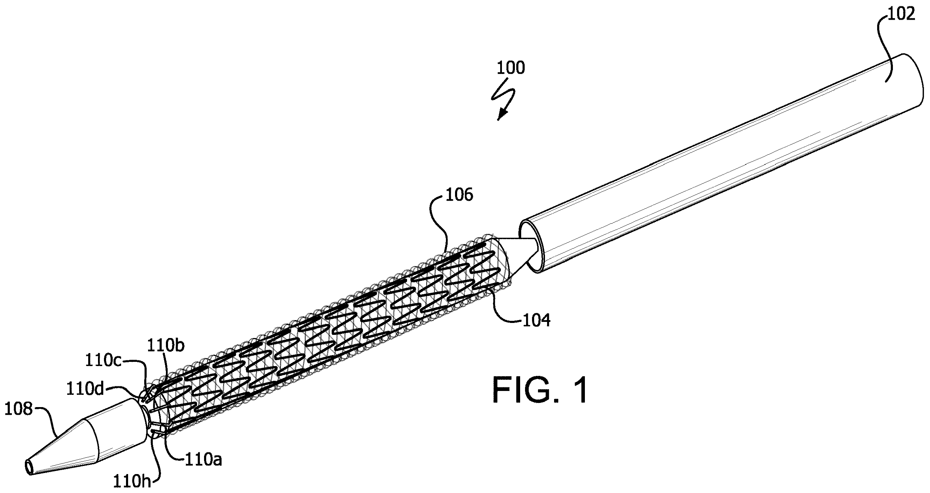

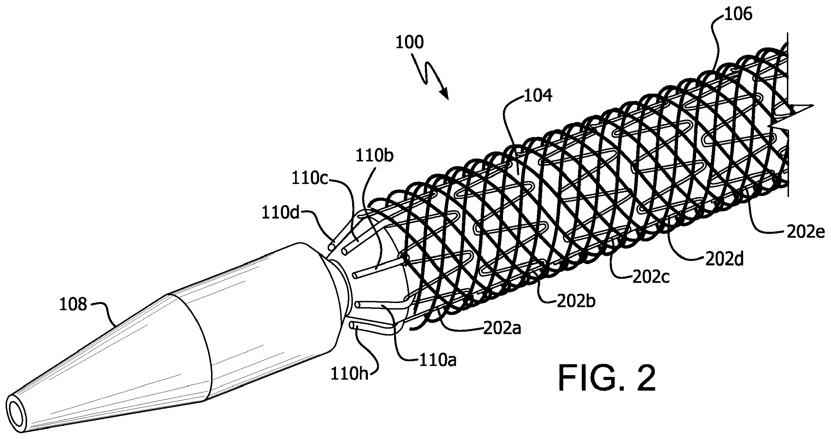

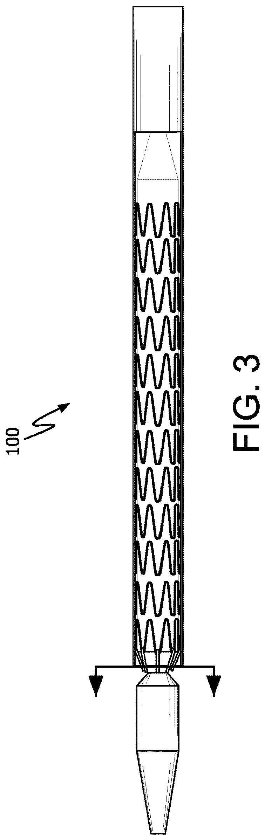

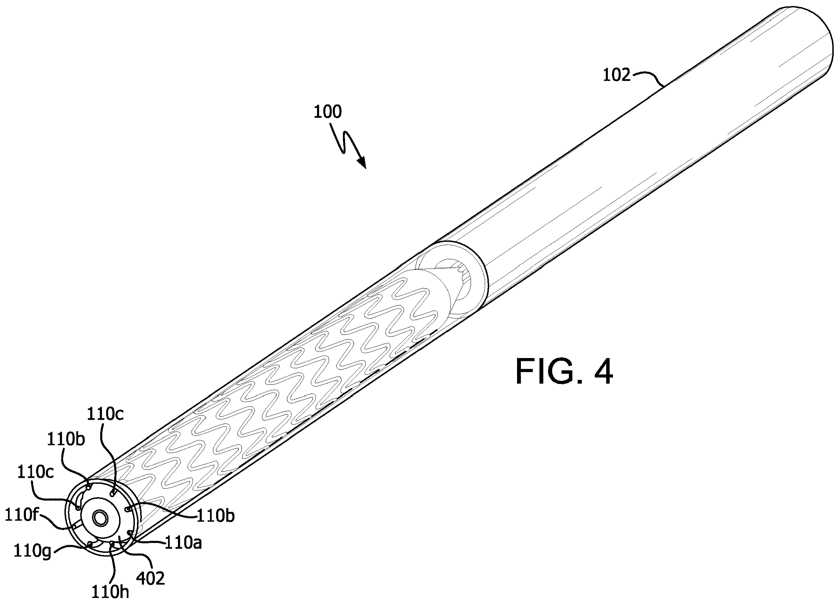

Now, as shown with reference to Figure 1, and more closely with reference to Figure 2, an endoprosthesis delivery system 100 can comprise, in various embodiments, an elongate member 102, an endoprosthesis 104, a covering member 106, and/or a catheter tip or "olive" 108. Further, as shown with reference to Figures 1-4 (and most clearly with reference to Figure 4, which as shown at Figure 3, illustrates a cross-sectional view of the delivery system 100), a delivery system 100 can comprise one or more flexible elements 110a-110h. In various embodiments, the distal edge of the covering member 106 can extend to a location just proximal of the ends of the flexible elements 110a-110h, for example, between the ends of elements 202a-202e (described below) and the distal ends of flexible elements 110a-110h. In certain embodiments, for example where covering member 106 comprises a plurality of woven warp knit or knit-braid fibers, the distal and/or proximal end regions of covering member 106 can be longitudinally compressed against the end(s) of endoprosthesis 104. This helps to reduce variability of such compression which might otherwise occur to a more random extent when the endoprosthesis delivery system 100 is passed through a hemostasis valve and/or when covering member 106 is deployed via a pull line.

Referring now mainly to Figure 2, each of the flexible elements 110a-110h can extend over, or overlap or overlay, a distal edge of the endoprosthesis 104. For example, the endoprosthesis 104 can comprise a stent or stent graft, which can, in either case, include one or more stent elements 202a-202e, e.g., stent rings or windings. Each element 202a-202e can comprise a plurality of apices, and, with regard to a stent element located at a distal end (or a proximal end, in certain embodiments) of the endoprosthesis 104, such as stent element 202a, the flexible elements 110a-110h can overlap the apices formed in the stent element 202a. Similarly, where the endoprosthesis 104 comprises a stent graft, the flexible elements 110a-110h can overlay the distal edge, e.g., an apex, and optionally extend through the graft material by way of one or more openings formed (e.g., via a laser cutting tool) in the graft material.

While the figures show eight flexible elements, in various embodiments, the number of flexible elements can correspond to a number of apices associated with an endoprosthesis 104. Thus, there can be fewer or greater than eight flexible elements 110a-110h. Moreover, two or more flexible elements can overlay a single apex.

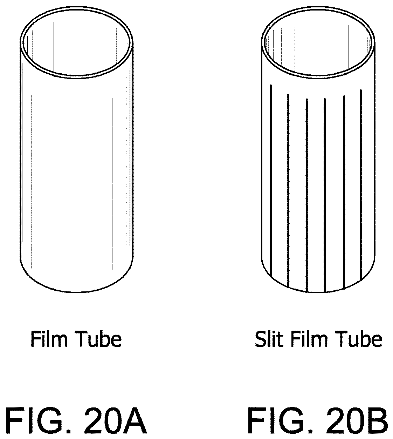

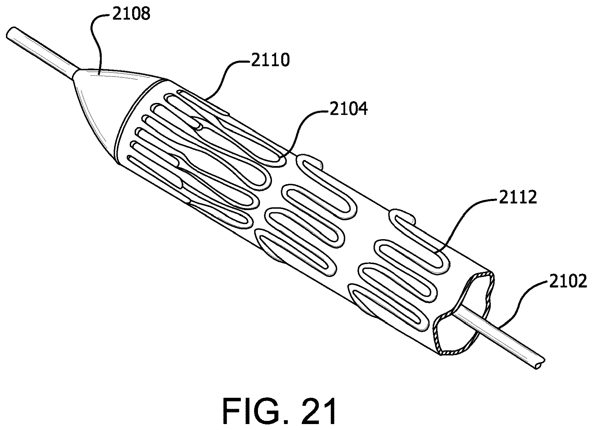

In various embodiments, and with reference to Figures 20A and 20B, a flexible element 110a-110h can be formed by cutting or incising a film tube to create a plurality of slits within the film tube. A film tube having a plurality of slits is shown with respect to Figure 20B, and as shown, the region between each slit can comprise one of the plurality of flexible elements 110a-110h. Similarly, as shown in Figure 21, in other embodiments, the flexible element(s) 2110 formed from a film tube having a plurality of longitudinal slits shown in Figure 20B can be secured to the distal region of the elongate member 2102, distal to the distal edge of the endoprosthesis 2104. For example, one or more flexible elements 2110 can be secured to a catheter olive 2108, or be positioned proximal thereto and extend proximally to overlay the edge of the endoprosthesis 2104, and optionally extend through openings formed adjacent to apices of the stent elements 2212.

As shown with respect to Figure 4, the endoprosthesis delivery system 100 can further comprise a material 402 compressible in the z-axis, e.g., a material that comprises some loft or a soft material, such as a silicone, ePTFE, or polyurethane foam. The "z-axis" can refer to the axis which extends through the thickness of the material. The compressible material 402 can surround the elongate member 102. The z-axis compressible material 402 extends on a section of the elongate member, wherein the section is collocated at an edge of the endoprosthesis 104 and extends at least a small distance distally and/or proximally so that the edge of the tubular endoprosthesis, whether a proximal and/or distal edge, can at least partially embed itself into the compressible material 402. This can serve to prevent the covering member becoming entangled with the endoprosthesis from the underside thereof.

As such, the compressible material 402 can comprise any material in which an endoprosthesis 104 can be at least partially embedded when in a delivery configuration. For example, the compressible material 402 can be capable of compressing and/or densifying in response to the application of a radially compressive force, as described above. In various embodiments, the compressible material 402 can comprise any of the following and/or any combination of the following substances: silicones, polyurethane, polytetrafluoroethylene (PTFE), porous polymers, such as expanded polytetrafluoroethylene ("ePTFE"), fluorinated ethylene propylene (FEP), various foams, any polymeric material, any fluoropolymer based material, and the like. The compressible material may be in the form of a balloon, of complaint, semi-compliant, or non-compliant type. When slightly inflated, the balloon will serve the function of the compliant material. In addition, a balloon that is slightly longer than the endoprosthesis 104 can form one or more compliant olives at one or both ends of the endoprosthesis 104. Further, in various embodiments, a covering member 106 can be used to adjust the length of a balloon at the end of the endoprosthesis 104.

In various embodiments, the flexible element(s) 110 can comprise a compliant member. The compliant member can be sufficiently compliant to conform to a distal edge profile of an endoprosthesis. The flexible element(s) 110 can further be a slender, elongated member. For example, flexible element(s) 110 can comprise a filament or thread. The flexible element(s) can possess any variety of cross-sections, e.g., a circular, oval, square, elliptical, rectangular, polygonal, generally flat, or any variety of cross-sections. Moreover, in various embodiments, the flexible elements 110 can comprise a tube, tube like structure, or sock. The flexible element(s) can be manufactured by methods typically used to make fibers and the like and by injection molding, extrusion, and/or calendaring.

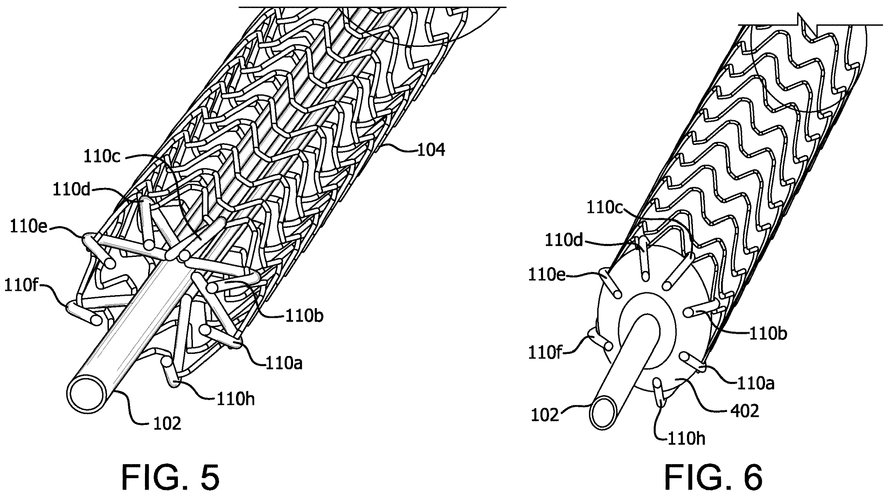

A flexible element is secured to the elongate member 102. For example, a flexible element 110 can be coupled to elongate member 102 at a location proximal to the distal edge and extend through an opening to overlay the distal edge, as shown with reference to Figure 5. The flexible element in this example is of an appropriate length so that the free end (the end not secured to the elongate member 102) is drawn out of the opening in an apex formed in the stent ring or winding as the endoprosthesis 104 deploys.

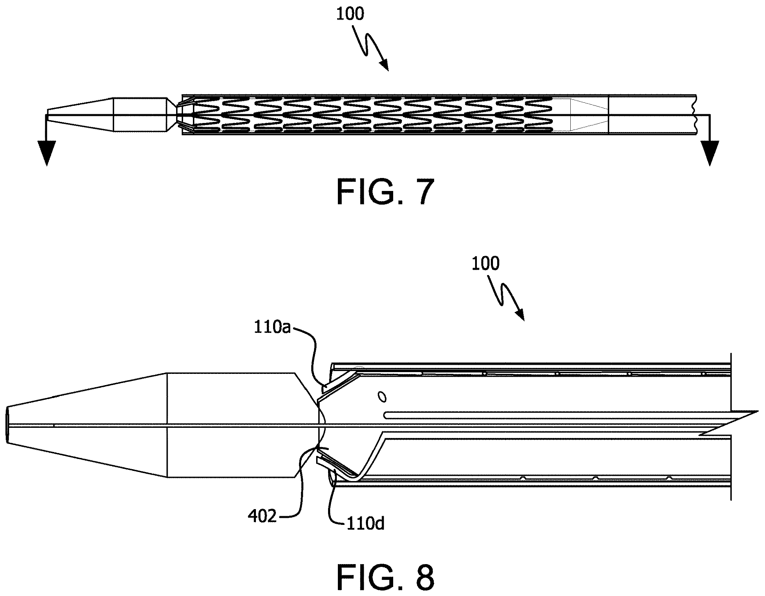

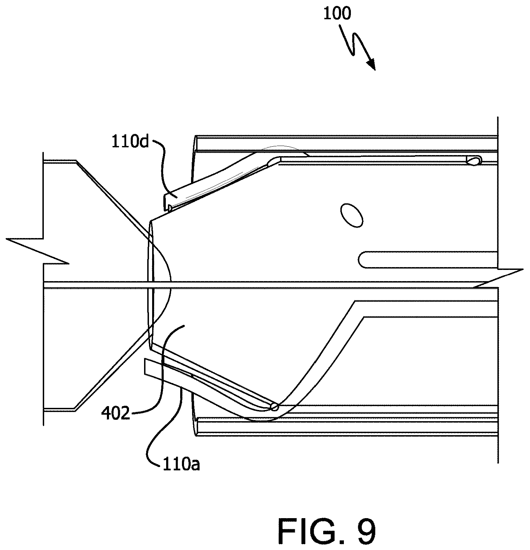

In order to secure the flexible element, as shown with respect to Figure 6, each flexible element 110a-110h can optionally extend into or beneath the compressible material 402. The flexible elements 110a-110h can, in this manner, be secured to the elongate member as each element extends proximally there under. This feature is illustrated in additional detail with respect to Figures 7-9. In particular, Figures 8 and 9 show cross-sectional views, taken along the line shown in Figure 7, of an endoprosthesis delivery system 100. In these Figures, several flexible elements 110a and 110d can be seen overlapping their respective apices and extending in a proximal direction underneath the surface of the compressible material 402.

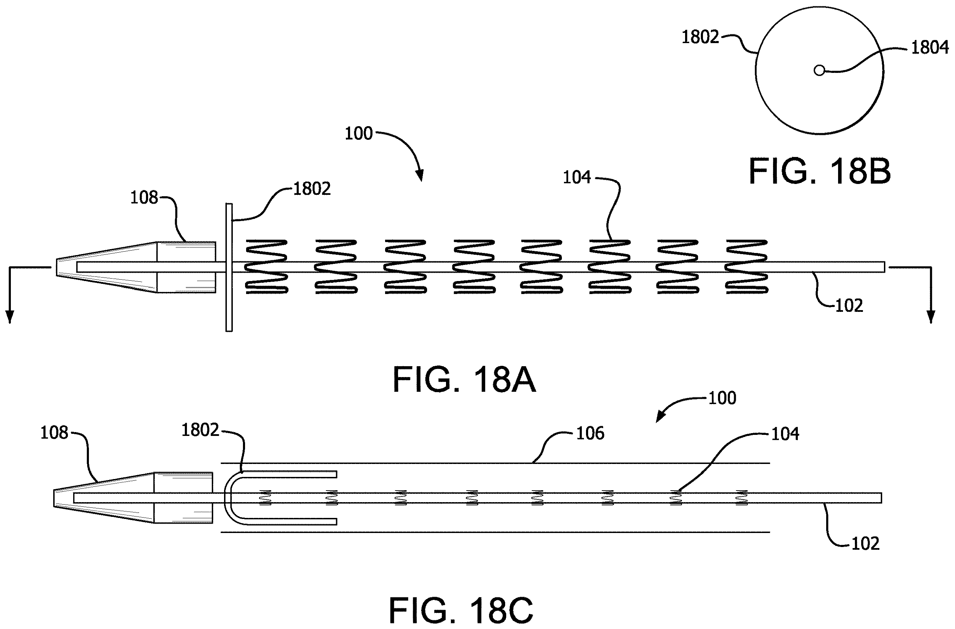

As shown in Figures 18A-18C, in other embodiments, the flexible element 110 can be formed as a thin, flexible disc 1802 which when attached to a catheter olive 108 or when positioned just proximal thereto as shown can be everted toward the endoprosthesis 104 to cover at least a portion of its edge. Figure 18A shows the delivery system 100 with a distal olive 108, elongate member 102 and endoprosthesis 104 all in an unconstrained state, e.g., before a covering member 106 is applied. A front view of disc 1802, having a center hole 1804 for fitting with elongate member 102 is shown in Figure 18B. Disc 1802 is shown in Figure 18C as having been everted over the distal edge of endoprosthesis 104 and covering member 106 applied to constrain both endoprosthesis 104 and disc 1802. Upon removal of covering member 106, disc 1802 prevents covering member 106 from becoming caught on the distal edge of endoprosthesis 104 and as endoprosthesis 104 expands, the edges of disc 1802 are rotated outward. Disc 1802 can be made of any sufficiently flexible material, e.g., an elastomer such as silicone or thin sheet of a polymer such as ePTFE, so that the readily rotates away from the endoprosthesis 104. Disc 1802 can be made of different shapes of any sufficiently flexible material, e.g. a sheet, tube of circular cross section or other non uniform cross sections.

In further embodiments, disc 1802 may be provided with longitudinal weak zones or perforations which fracture upon expansion of endoprosthesis 104. In other embodiments, disc 1802 may feature a series of longitudinal cuts made around its circumference extending radially toward, but not extending to, the center hole 1804. Such cuts form a plurality of strands about at least a part of the disc's circumference. These strands can then be everted over the distal end of endoprosthesis 104 and serve to prevent covering member 106 from entangling with the distal edge thereof upon its removal.

At the point of attachment, flexible element can easily pivot or bend at its base, i.e., the point of catheter attachment, facilitating the flexible element sweeping through a wide range of motion. For example, the flexible element is capable of sweeping through at least a 90 degree arc along the longitudinal axis, at least a 180 degree arc along the longitudinal axis, at least a hemispherical range, or more.

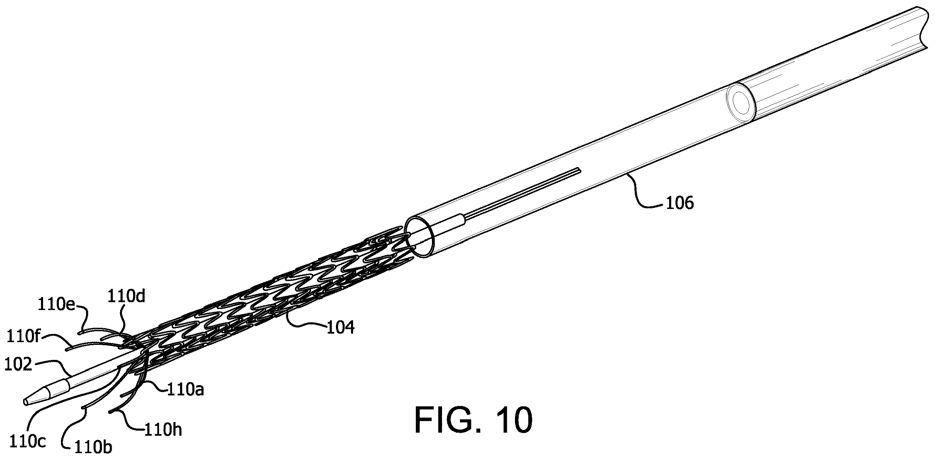

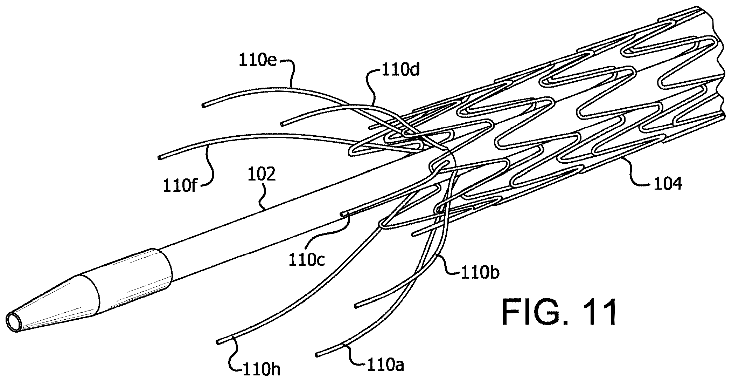

With reference now to Figures 10 and 11, an endoprosthesis 104 can be delivered as shown. The covering member 106 can be unstitched, unzipped, unraveled, drawn away, or otherwise removed, as described herein, from the endoprosthesis 104. Covering member 106 is shown as being fully retracted for clarity but it will be understood that endoprosthesis 104 can progressively expand during gradual or stepwise withdrawal of covering member 106. As the covering member 106 is removed from an end of the endoprosthesis 104 (e.g., as depicted, from a distal end or region of the endoprosthesis 104), the flexible elements 110a-110h guide the retracting covering member 106 over the distal edge, e.g., at the apices, at the distal (or proximal, in other configurations) end of the endoprosthesis 104. As the covering member 106 uncovers the tubular endoprosthesis, the flexible elements 110a-110h can be pushed outward by the expanding endoprosthesis, and optionally retracted from an opening. This feature can be of particular help where, for example and as described above, the covering member 106 is of a braided or knitted structure, such a structure having openings capable of becoming entangled snagging, or catching on one or more apices as the covering member 106 is made to unzip through the proximal motion of a pull line coupled to the member 106.

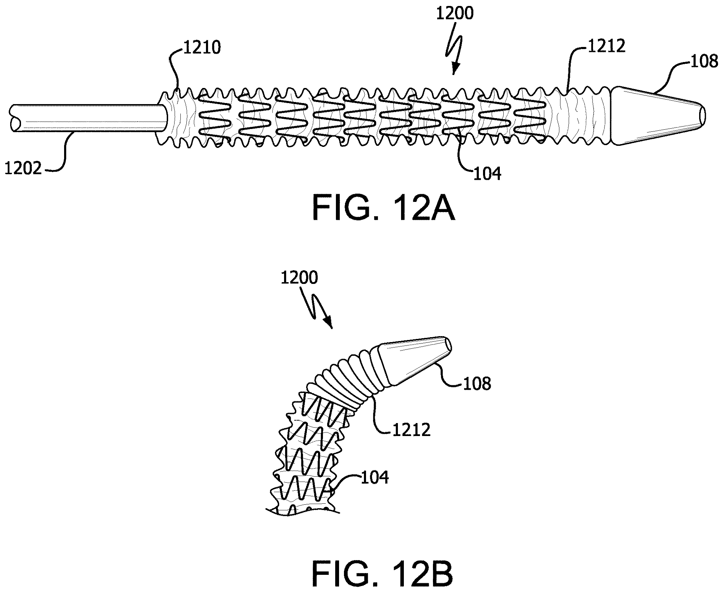

In various embodiments, and referring to Figure 12A, an endoprosthesis delivery system 1200 can include an elongate member, an endoprosthesis 104, a covering member, as described above, and a slip resistant portion or material 1210 located about a section of catheter adjacent the endoprosthesis 104.

In various embodiments, the slip resistant material 1210 can be located on the elongate member 1202 adjacent to and/or underneath the endoprosthesis. As shown in Figure 12 A, slip resistant material 1210 is positioned proximal of the olive 108, extending under endoprosthesis 104 and terminating proximal thereto. In various embodiments, slip resistant material may extend a longer or shorter distance, e.g., it may extend proximal of the olive 108 to just under the distal edge of endoprosthesis 104 or abut the distal edge of the endoprosthesis.

In various embodiments, the slip resistant material 1210 can comprise a z-axis compressible material, as described above. In same or different embodiments, the slip resistant material 1210 can be longitudinally compressed, as shown, so that its surface is not smooth, but wrinkled or ribbed, giving it a more "abrasive" surface to resist bunching of the covering member. Further, in same or different embodiments, the slip resistant material 1210 can be gummy or mildly sticky or adhesive. Slip resistant material can be made of a variety of materials such as adhesives, materials coated with adhesives, spongy materials possessing loft, foams, or materials that are compressible. One such material can be made of a polymer such as PTFE or an ePTFE. In certain embodiments, slip resistant material 1210 may be incorporated with or serve as the olive 108. In such embodiments, the distal end of the endoprosthesis 104 will extend over at least a portion of the olive 108. Where slip resistant material 1210 forms the olive 108, said material can comprise a foam.

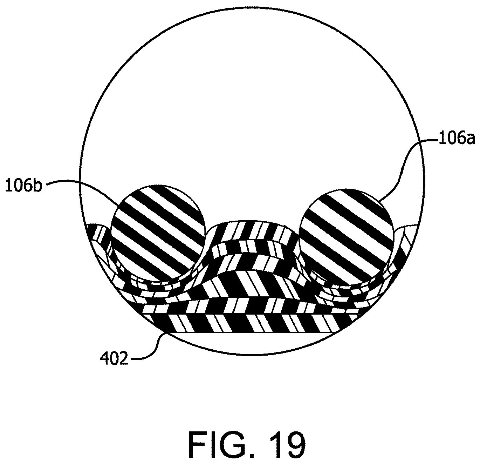

During deployment, the covering member (not shown) can be removed, e.g., by unraveling or unzipping, to release the endoprosthesis 104 from a constrained configuration. In various embodiments, as the covering member unzips, the covering member can stick to and/or at least partially embed into the slip resistant material 1210, for example in a distal portion or segment 1212 of the slip resistant material 1210, so that the covering member is impeded from bunching as a pull line coupled to the covering member is pulled to deploy or uncover the endoprosthesis. A cross-sectional illustration of a covering member embedded within a slip resistant or otherwise compressible material is shown at Figure 19. With reference to Figure 19, as shown, one or more sheath or covering member fibers 106a and/or 106b can be embedded within a compressible material 402. This can, in turn, prevent, or help to prevent a snag or entanglement between the covering member and the endoprosthesis 104. In various embodiments, slip resistant material 1210, in particular segment 1212, can function to elongate along the outer radius when bent and thus minimizing a gap formed between the proximal face of olive 108 and the distal edge of the endoprosthesis 104. If the covering member extends proximally or over olive 108, segment 1212 will prevent covering member from slipping into said gap. If this occurs, covering member has an increased likelihood of becoming caught on the endoprosthesis 104.

With particular attention to the interface between the edge of the endoprosthesis 104 and the covering member, as the pull line coupled to the covering member is retracted, the covering member, as it is removed proximally, can be impeded by its bond with the segment 1212 of slip resistant material 1210 from sliding down what might otherwise comprise a relatively smooth surface of the elongate member, bunching in the process, to eventually become entangled with an end of the endoprosthesis 104. Thus, the segment 1212 of slip-resistant material 1210 can prevent or reduce entanglement of the covering member with an end of the endoprosthesis 104.

In addition, in a further embodiment, the segment 1212 of slip resistant material 1210 can extend underneath the edge of the endoprosthesis, and the edge of endoprosthesis can be at least partially embedded into the segment 1212 of slip-resistant material 1210. For example, one or more apices at the edge of the endoprosthesis 104 can be embedded into or nested within the folds of the slip resistant material. Therefore, as the covering member is removed, the member can simply ride over the end of the endoprosthesis 104, and this can further reduce entanglement between the end of the endoprosthesis 104 and the covering member. Further still, in certain embodiments, the segment 1212 of the slip resistant material 1210 can act to share the load with the endoprosthesis 104. For example, as the endoprosthesis is maneuvered through the anatomy of a patient (particularly, tortuous anatomy), the segment 1212 of slip resistant material 1210 can permit the endoprosthesis delivery system 1200 to navigate through the anatomy more gently, e.g., by turning around curves and corners more subtly and/or gently.

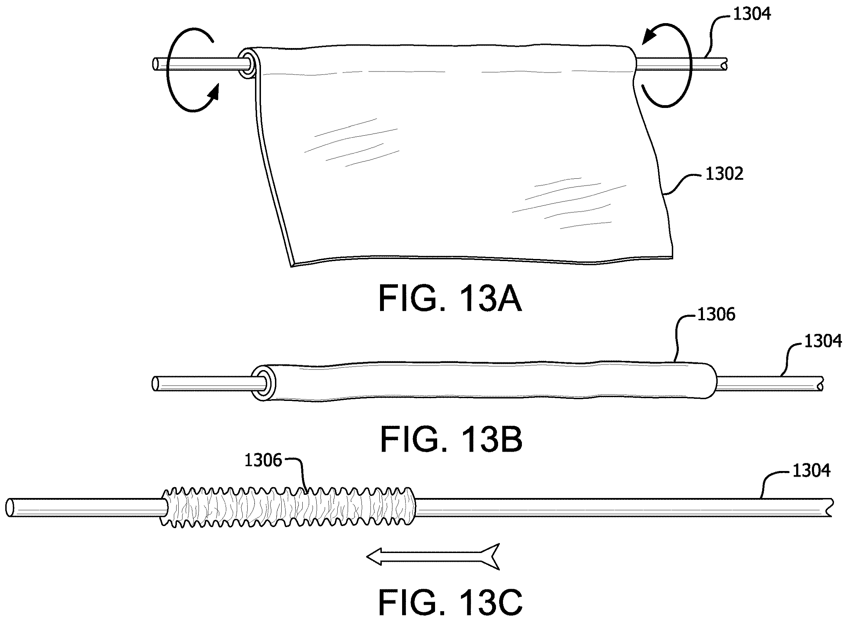

A slip resistant material 1210 can be manufactured, in various embodiments, as shown at Figures 13A-13C. In particular, as shown at Figure 13A, a sheet or tape of compliant material 1302 can be wound or rolled about a mandrel 1304. In various embodiments, compliant material 1302 can comprise a sheet or tape of thin flexible polymeric sheet and optionally, a porous polymer, such as an ePTFE membrane. Now, as shown with reference to Figure 13B, the rolled sheet 1306 can be treated, e.g., thermally treated, to prevent it from unrolling and/or adhere it together. Alternatively or in addition thereto, the rolled sheet can be cured with a flexible and optionally an elastomeric adhesive, such as a thermoplastic copolymer of tetrafluoroethylene and perfluoroalkylvinylether as described in U.S. Patent No. 8,048,440 entitled "Thermoplastic Fluoropolymer-Coated Medical Devices" and U.S. Patent No. 7,462,675 entitled "Thermoplastic Copolymer of tetrafluoroethylene and perfluoromethyl vinyl ether and Medical Devices Employing the Copolymer,". The rolled sheet 1306 can finally be scrunched or compressed about its longitudinal axis, as shown at Figure 13C, to form the slip resistant material 1210. In addition, in various embodiments, a slip resistant material 1210 can also be manufactured by helically wrapping a sheet of compliant material 1302 over a mandrel and/or sliding a tube comprising such a material 1302 over an elongate member.

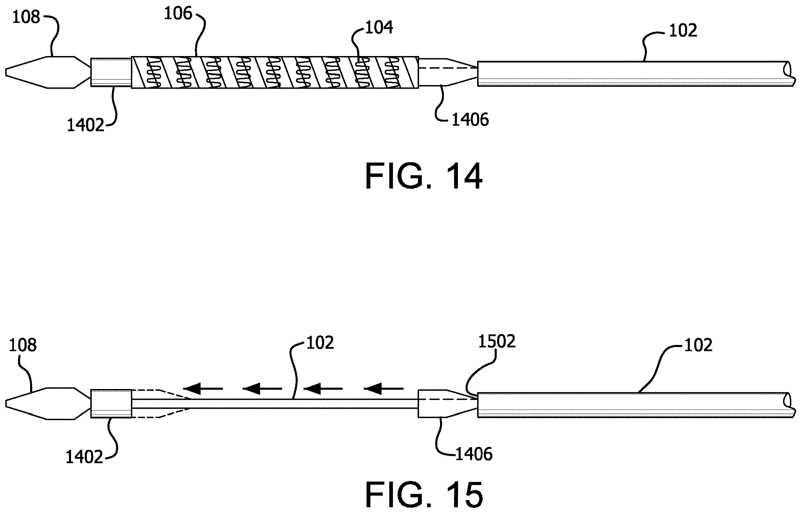

Referring now to Figure 14, an endoprosthesis delivery system 1400 is shown. The delivery system 1400 can comprise, in various embodiments, an elongate member 102 is contained within a concentric elongate member 1504 such as an introducer sheath , an endoprosthesis 104, a covering member 106, an end cap 1402 adjacent the edge of the endoprosthesis, and optionally, a tapered end cap 1406. The end cap 1402 prevents the covering member from hanging on the endoprosthesis. In various embodiments, the gap or space, if any, between the end cap 1402 and the edge of the endoprosthesis is less than the diameter of a flexible element. More preferably, the gap between the end cap 1402 and the edge of the endoprosthesis when extended along a curve having a radius commonly encountered in the vasculature is less than the diameter of a flexible element. As such, the end cap 1402 can prevent the endoprosthesis from snagging on the edge of the endoprosthesis.

The tapered end cap 1406 increases in diameter in a proximal to distal direction. In various embodiments, as discussed in greater detail below, the tapered end cap 1406 can further comprise one or more raised surface features on the widest or largest diameter portion of the tapered end cap 1406. These raised surface features can frictionally engage with the inner edge of the concentric elongate member 1504 so that the elongate member 1504 can slide through, but upon advance and contact with the end cap 1406, an increased pull on the elongate member can allow the tapered end cap 1406 through the concentric elongate member 1504. For example, the raised surface features can be a deformable material to facilitate passage through concentric elongate member 1504.

In various embodiments, the end cap 1402, when located on a curved section of vasculature, can optionally bend and elongate along the outer radius. For example, some bending can be facilitated by the end cap 1402 that is constructed of a low durometer tubular piece, as described herein. In other embodiments, the end cap 1402 can comprise a longitudinally compressed polymeric material having creases and folds and abutting the edge of the endoprosthesis and optionally partially deforming thereto, similar to that illustrated in Fig. 12B. The creases and folds provide some stored length that can be stretched when on a curve.

In other embodiments, the end cap 1402 can comprise a small inflatable member that presses against the edge of the endoprosthesis. In other embodiments, the end cap 1402 can comprise an open or closed cell polymeric foam material that presses against the edge of the endoprosthesis, the foam comprising a premanufactured component or being formed in place.

As described herein, the endoprosthesis 104 can have a distal edge as well as a proximal edge. As shown, the distal edge can abut or be disposed adjacent to the end cap 1402. Similarly, as shown, the proximal edge can abut or be disposed adjacent to the tapered end cap 1406. Further, as shown, the diameter of the constrained endoprosthesis 104 can be substantially the same as the diameter of the end cap 1402 and/or the diameter of the distal portion of the tapered member 1406.

With particular regard to the compositions of the end cap 1402 and optionally the tapered end cap 1406, the components can comprise all or in part, any of a variety of materials. For instance, both components can comprise a mildly or moderately deformable material, i.e., a low durometer material at least on the section closest to the endoprosthesis. For example, the low durometer material can have a durometer between 15 and 70 Shore on the Type A scale. In various embodiments, the end cap 1402 can be constructed so as to more compliant along the axis parallel to the longitudinal axis of the guiding member than across its radial dimension.

In various embodiments, the end cap 1402 can be at least partially deformed to the edge profile of the endoprosthesis 104. This can be accomplished by way of any suitable process and in any manner that is suitable for the purpose. For instance, the proximal edge of the end cap 1402 can be pressed against the distal end of the endoprosthesis 104, and the deformable properties of the end cap 1402 (or at least the proximal end thereof), can permit the end cap 1402 to at least partially deform to the edge of endoprosthesis 104. In addition or alternatively, in various embodiments, the end cap 1402 can comprise a concave proximal end, so that the thinnest section of the end cap has the largest diameter. Having a thinner section can facilitate improved deformation to the edge profile.

With further regard to the end cap 1402, at its distal end, the end cap 1402 can optionally be coupled to a catheter olive 108. The end cap 1402 can be coupled to the olive 108 in any suitable manner. For example, the distal end of the end cap 1402 can fit into a receiving portion formed in the proximal end of the olive 108 and/or bonded within the receiving portion in any suitable fashion, including any number of suitable techniques and/or using any of the materials described herein (e.g., heat bonding, radio frequency bonding, pressure bonding, glue or adhesives, and the like).

The covering member 106 can extend over at least a portion of the end cap 1402 and beyond the end cap 1402. Likewise, in various embodiments, the covering member 106 can extend over at least a portion of the tapered end cap 1406.

During deployment, the covering member 106 can be removed (e.g. unraveled) to uncover or release the endoprosthesis 104 from a constrained diameter. As this process occurs, the end cap 1402 can prevent the covering member 106 from becoming caught, snagged, or entangled with the edge of the endoprosthesis 104. Rather, the end cap 1402 guides the covering member 106 over the edge of the endoprosthesis 104. The same process can occur at the proximal end of the endoprosthesis 104, except that the covering member 106 can follow the tapered end cap 1406 over the proximal end of the endoprosthesis 104. (Optionally, the end cap 1402 can be a mirror image of the tapered end cap 1406; i.e., its tapered end increases in diameter from a distal to proximal direction.)

In various embodiments, a post-deployment state can take place after and/or in response to deployment of the endoprosthesis 104. A post-deployment state is illustrated at Figure 15. As shown, the endoprosthesis 104 has been deployed, exposing the elongate member 102. The elongate member 102 can be coupled to the end cap 1402 and the tapered end cap 1406 through a channel formed within each component. The tapered end cap 1406 can be slideable along the elongate member 102 and have a distal face that is substantially the same cross-sectional area and shape as the proximal face of the end cap 1402.

To remove the delivery system 1400 from the body lumen of a patient, a physician can withdraw, or cause to be withdrawn, the elongate member 102. As this occurs, the elongate member 102 can retract proximally through the introducer sheath or other concentric elongate member 1504. The tapered end cap 1406, which is slideable along the elongate member 102 and has a distal cross-sectional area substantially equal to or slightly more than the inner diameter of the introducer sheath 1504, can remain in place against an open end 1502 of the introducer sheath 1504 as the elongate member 102 continues to be withdrawn. Thus, as the elongate member 102 is withdrawn, the proximal face of the end cap 1402 can approach the distal end of the tapered end cap 1406 until contact is made. As contact is made, the end cap 1402, which is frictionally engaged or otherwise secured to the elongate member 102 can act as a backstop to the tapered end cap 1406, and, at this juncture, with an additional pull force applied by the operator, the tapered end cap 1406 can be pulled through the open end 1502 of the concentric elongate member 1504. The tapered end cap 1406 can thus facilitate retraction of the end cap 1402 (which can have a substantially non-tapered proximal face) into the open end 1502 of the outer shaft of the concentric elongate member 1504. Absent the tapered end cap 1406, the end cap 1402 might hang on the open end 1502 as an attempt is made to retract the elongate member 102 back through the concentric elongate member 1504, particularly if the open end 1502 is located on a curve in the vasculature and the elongate member 102 is entering the open end 1502 at an angle relative to the tangential axis at the open end 1502.

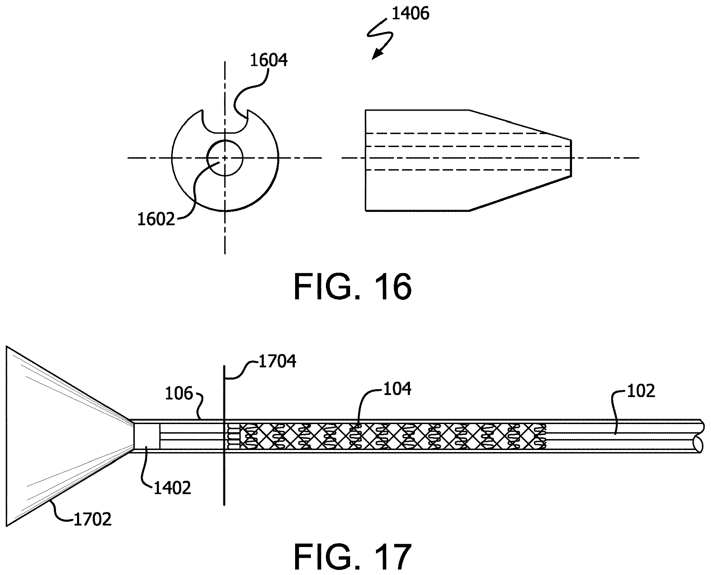

With continuing attention to the tapered end cap 1406, and with brief reference to Figure 16, a channel 1602 is shown formed within the end cap 1406. This channel can accommodate, as discussed above, the inner shaft 102. In addition, a concavity or depression 1604 is shown, which can accommodate a pull line for reduced profile and it is contemplated that a plurality of depressions or other features can exist predicated on system needs.

With reference to Figure 17, in various embodiments, the endoprosthesis 104 can be loaded with the end cap 1402 and the covering member 106 using a loading funnel 1702. In particular, the endoprosthesis 104 can be reduced from an unconstrained diameter to a constrained diameter by feeding the endoprosthesis 104 through the loading funnel 1702. The constrained endoprosthesis 104 can be received, at the reduced diameter portion of the loading funnel 1702, by the covering member 106. An inner elongate member 102 can extend substantially coaxially through the covering member 106, and as the endoprosthetic device 104 is fed through the funnel 1702 into the covering member 106, the endoprosthesis 104 can further substantially coaxially straddle the inner elongate member 102.

Having received the endoprosthesis 104, the covering member 106 can be cut or otherwise severed at a particular location. The location can be along a cutline 1704, and the cutline 1704 can be, as shown, substantially adjacent to, but slightly distal of, the distal end of the endoprosthesis 104. However, care should be taken not to sever the inner elongate member 102 during this process. Further, although a particular cutline 1704 is shown, in various embodiments, the covering member 106 can be severed at any location along the length of the endoprosthesis 104 and/or at any location(s) distal and/or proximal of the endoprosthesis 104. Having established the cutline 1704, any excess covering member 106 can be removed.

In the absence of the excised covering member 106, the end cap 1402 can be coupled to the exposed elongate member. More particularly, the end cap 1402 can, in various embodiments, be mounted on the elongate member and advanced along the elongate member until the end cap 1402 abuts or is compressed against the distal or proximal edge (e.g., the distal apices) of the endoprosthesis 104. The end cap 1402 can be coupled to the elongate member in any manner suitable for this purpose, such as by way of any of the techniques and/or materials described herein. For example, the end cap 1402 can be frictionally engaged to the elongate member, or be glued or otherwise adhesively fixed to the elongate member. Further, the end cap 1402 can be heat bonded, radio frequency bonded, pressured fitted or bonded, and the like to the elongate member 102.

The tapered end cap 1406 can be similarly coupled to the elongate member 102. For instance, the covering member 106 can be removed or excised from a portion of the elongate member 102 proximal to the endoprosthesis 104 and the tapered end cap 1406 mounted on the elongate member 102 by way of the channel 1602 formed in the member 106. The tapered end cap 1406 can be advanced along the elongate member 102 until the tapered end cap 1406 abuts or is compressed against the proximal end of the endoprosthesis 104. The tapered end cap 1406, however, may not be coupled to the elongate member 102, because, as described above, the tapered end cap 1406 can be slideably coupled to the elongate member 102.

In various embodiments, the features and advantages associated with each of the endoprosthesis delivery systems 100, 1200, and 1400, as described above, can be combined in any desirable manner. For example, although the system 100 is not described above as inclusive of a tapered end cap 1406, the system 100 can, in various embodiments, include such a tapered end cap 1406. Similarly, although the compressible material 402 is not described, with respect to system 100 as being folded or ribbed, as disclosed with respect to system 1200, the compressible material 402 can nevertheless comprise such a texture and/or conformation.

A graft comprising any of the grafts and/or stent-grafts described above may be made up of any material which is suitable for use as a graft in the chosen body lumen. A graft may comprise one or a variety of materials. Furthermore, a graft may comprise multiple layers of material, which can be the same material or different material. Although a graft may have several layers of material, the graft may have a layer that is formed into a tube (innermost tube) and an outermost layer that is formed into a tube (outermost tube). In some embodiments, a graft may be fenestrated with a fenestration tool.

Many graft materials are known, and in various embodiments, these materials can be used in combination and assembled together to comprise a graft. These materials may be further extruded, coated and/or formed from wrapped films, and/or a combination thereof. Polymeric materials, biodegradable materials, and/or natural materials can be used for specific applications.

In various embodiments, a graft may comprise synthetic polymers including nylon, polyacrylamide, polycarbonate, polyformaldehyde, polymethylmethacrylate, polytetrafluoroethylene, polytrifluorochlorethylene, polyvinylchloride, polyurethane, elastomeric organosilicon polymers, polyethylene, polypropylene, polyurethane, polyglycolic acid, polyesters, polyamides, their mixtures, blends, and copolymers. In a variety of embodiments, a graft may be made from a class of polyesters such as polyethylene terephthalate including DACRON^{®} and MYLAR^{®} and polyaramids such as KEVLAR^{®}, polyfluorocarbons such as polytetrafluoroethylene (PTFE) with and without copolymerized hexafluoropropylene (TEFLON^{®} or GORE-TEX^{®}), and porous or nonporous polyurethanes. Further, in a variety of embodiments, a graft may comprise expanded fluorocarbon polymers (especially PTFE) materials.

In various embodiments, fluoropolymers, generally referred to herein, may include polytetrafluoroethylene (PTFE), expanded PTFE (ePTFE), fluorinated ethylene propylene (FEP), copolymers of tetrafluoroethylene (TFE) and perfluoro (propyl vinyl ether) (PEA), homopolymers of polychlorotrifluoroethylene (PCTFE), and its copolymers with TFE, ethylene-chlorotrifluoroethylene (ECTFE), copolymers of ethylene-tetrafluoroethylene (ETFE), polyvinylidene fluoride (PVDF), and polyvinylfluoride (PVF). In various embodiments, a graft can comprise any combination of the materials listed above. Further, in various embodiments, a graft may be substantially impermeable and/or permeable to bodily fluids. A substantially impermeable graft may be made from materials that are substantially impermeable to bodily fluids or can be constructed from permeable materials treated or manufactured to be substantially impermeable to bodily fluids (e.g. by layering different types of materials described above or known in the art). In various embodiments, a stent-graft and/or a side-branch stent-graft, as described above, may be made from any combination of the materials described above, including ePTFE.

Any stent, including stent and/or stent members may be generally cylindrical when restrained and/or when unrestrained and may comprise helically arranged undulations having a plurality of helical turns or wraps. In a variety of embodiments, undulations may be aligned so that they are "in-phase" with each other. More specifically, undulations may comprise apices in opposing first and second directions. When these undulations are in-phase, apices in adjacent helical turns are aligned so that apices can be displaced into respective apices of a corresponding undulation in an adjacent helical turn. In certain embodiments, undulations may have a sinusoidal shape, a U shape, a V shape, and/or an ovaloid shape.

In various embodiments, a stent may be fabricated from a variety of biocompatible materials including commonly known materials (or combinations of materials) used in the manufacture of implantable medical devices. Such materials may include 316L stainless steel, cobalt-chromium-nickel-molybdenum-iron alloy ("cobalt-chromium"), other cobalt alloys such as L605, tantalum, nitinol, polymers, biodegradable alloys or polymers, or other biocompatible metals. In some embodiments, any stent and/or stent-graft described herein may comprise a balloon expandable stent and/or stent-graft and/or a self-expanding stent and/or stent-graft. Further, in certain embodiments, a stent may comprise a wire wound stent, which may or may not comprise undulations.

### Example 1 - Delivery System Construction

In various embodiments, an endoprosthesis delivery system 100 can be manufactured and/or constructed as follows. Generally, an inner shaft 102 wrapping procedure can be followed by an endoprosthesis loading procedure. The inner shaft 102 wrapping procedure can begin with the insertion of a processing mandrel and/or an appropriate inner shaft 102 within a pair of processing chucks coupled to a film wrapping device. The wrapping device can comprise a film payoff head, which can be configured to travel along a length of the inner shaft 102 to wrap a tape or other compressible or compliant material, as described herein, about the shaft 102. Thus, the inner shaft 102 can be wrapped with a compliant material by the wrapping device. In various embodiments, the film payoff head can make a first pass along an axial length of the shaft 102 and a second pass, in the reverse direction, along the shaft 102 as well. However, the film payoff head can, in other embodiments, make as many or as few passes over the shaft 102 as are desired.

One or more flexible elements 110a-110h can, in addition and as described herein, be coupled to the inner shaft 102. More particularly, in various embodiments, one or more flexible elements 110a-110h can be attached or adhered, in any suitable configuration, to the shaft 102 (e.g., over the layer of compliant material deposited, as discussed, above) and/or one or more runs or layers of film or other suitable compliant material layered over each element 110a-110h to bind the elements 110a-110h to the shaft 102. Thus, the inner shaft wrapping procedure can result in a shaft coupled to one or more elements 110a-110h and/or one or more layers of compliant material.

An endoprosthesis 104 can, as part of an endoprosthesis loading procedure, be further loaded on a shaft 102. The shaft 102 can, in various embodiments, be coupled to the one or more flexible elements 110a-110h prior to the loading procedure. Therefore, to load the endoprosthesis onto the shaft 102, an unconstrained endoprosthesis 104 can be inserted over the shaft 102 and fed, in this configuration, into a loading funnel. In addition, as the endoprosthesis 104 enters the loading funnel, each of the flexible elements 1410a-110h can be threaded, as described herein, through one or more of the apices of the endoprosthesis 104. The loading funnel can, in this manner, reduce the endoprosthesis 104 to a constrained diameter about the shaft 102. A covering member 106, as described herein, can be further positioned at the smaller diameter end of the loading funnel, and the constrained endoprosthesis 104 can be thus fed into the covering member 106 at the output of the funnel.

### Example 2 - Delivery System Construction

Continuing, in various embodiments, an endoprosthesis delivery system can be constructed and/or manufactured with an end cap 1402 and/or a tapered end cap 1406 as follows. An endoprosthesis 104 can, as described herein, be loaded through a loading funnel. A process for loading an endoprosthesis through a loading funnel is also generally described by United States Patent No. 6,702,845 to Cully et al., issued March 9, 2004, entitled "Compacted implantable medical devices and method of compacting such devices". The endoprosthesis 104 can be loaded by insertion through the loading funnel on the inner shaft 102. The distal end of the endoprosthesis can pass through the funnel last, so that the proximal end of the endoprosthesis 104 is received, at the smaller diameter end of the funnel, and as described above, by the covering member 106.

At the distal end of the endoprosthesis 104, the end cap 1402 can be coupled to the distal edge of the shaft 102. For example, in various embodiments, the end cap 1402 can comprise any compliant, compressible, elastomeric materials, and the like, such as Shore A 45 durometer PEBAX, and having an inside diameter sized to fit slightly to the distal edge of the shaft by way of compression, interference, or impression between the shaft 102 and the end cap 1402. The outside diameter of the end cap 1402 can have, in various embodiments, a diameter slightly smaller than a diameter of the loading funnel. Thus, the end cap 1402 can be coupled through the loading funnel to the distal edge of the shaft 102. The end cap 1402 can be pressed against the distal edge to minimize any gap between the end cap 1402 and the distal edge of the shaft. Further, as discussed above, the endoprosthesis 104 can be advanced through the loading funnel and into a constrained configuration within the covering member 106. The end cap 1402 can be bonded to the distal edge of the shaft 102 by any suitable means, including, for example, using an adhesive and/or a melt bonding technique.

In like fashion, the tapered end cap 1406 can be coupled to the proximal edge of the shaft 202. For example, the tapered end cap 1406 can comprise any suitable material, including compliant, compressible, elastomeric materials and the like. More particularly, in various embodiments, the end cap 1406 can comprise a Shore A 40 durometer PEBAX. Further, in various embodiments, a radiopaque marker can be located in an end and/or proximate to an edge of the end cap 1406. The inside diameter of the end cap 1406 can, as discussed herein, be slidably coupled to the shaft 102, and prior to deployment, the tapered end cap 1406 can be positioned near the distal edge of the shaft 102.

### Example 3 - Delivery System Construction

In various embodiments, a small tubular extrusion of PEBAX can be obtained. The extrusion dimensions can be approximately 1 mm ID x 1.2 mm OD, and the material can have a Shore A hardness of 55. A stainless steel mandrel can be inserted into the extrusion lumen and gripped at both ends in a fixture to allow rotation of the extrusion. Further, an ePTFE film, as disclosed by United States Patent No. 5,814,405 to BRANCA, et al., issued September 29, 1998, entitled "Strong, air permeable membranes of polytetrafluoroethylene,", can be wrapped about the extruded tube and attached to the extrusion through thermal treatment, for example using a Weller soldering iron. A strip of film (e.g., in various embodiments, a strip of approximately 80mm in width) can be longitudinally wrapped about the extrusion parallel to the central axis of the extrusion, using minimal tension, by rotating the extrusion. Where such a strip is used, the 80mm width can be approximately 20 mm longer than the length of the medical implant (e.g., the endoprosthesis 104) being delivered; therefore, once the implant is centrally loaded on this compressible member, there will be approximately 10mm of compressible material exposed at either end of the implant. In various embodiments, differing numbers of film layers can be applied. For instance, in certain embodiments, approximately 30 layers of film can be applied, thus building up a lofty, compressible zone over which to load an endoprosthesis 104. In various embodiments, the compressible member can be formed in place using an appropriate mold and moldable material such as an injectable foam.

The film strip can then be cut and its loose end attached to an underlying layer, again through the use of localized heat. This compressible zone can then be used as is to load a stent, stent graft, or other endoprosthesis 104. During the loading process, the endoprosthesis 104 can be radially compressed into its delivery dimensions. During compression, the device compresses into the loft of the compressible member, which increases device to catheter retention quality, which ultimately improves accuracy of deployment in the clinical setting. The constraining device can also receive benefit since it too can compress into the compressible material which extends approximately 10mm at each end of the implant. This can assist in stabilizing it during subsequent delivery and deployment. Optionally, and to add increased benefit, the compressible material can have a layer of silicone (available from NuSil, Carpinteria, CA. as product # MED 1137) applied to it. Application can be accomplished by dipping, spraying or brushing or the like. Upon curing, this thin, tacky layer adds additional utility in that it can assist in adhering the layers of the ePTFE film together and can provide a tacky surface to which both endoprosthesis 104 and covering member 106 can temporarily grip during the delivery and deployment sequence.

Numerous characteristics and advantages have been set forth in the preceding description, including various alternatives together with details of the structure and function of the devices. The disclosure is intended as illustrative only and as such is not intended to be exhaustive. It will be evident to those skilled in the art that various modifications may be made, especially in matters of structure, materials, elements, components, shape, size, and arrangement of parts including combinations within the principles of the invention, to the full extent indicated by the broad, general meaning of the terms in which the appended claims are expressed. To the extent that these various modifications do not depart from the appended claims, they are intended to be encompassed therein.

## Claims

1. An endoprosthesis delivery system (1400) comprising:
an elongate member (102);
a tubular endoprosthesis (104) comprising a distal end and being disposed about the elongate member (102);
a first end cap (1402) having a distal end and a proximal end, and being positioned such that the proximal end of the first end cap (1402) is adjacent to the distal end of the endoprosthesis (104);
a second end cap (1406) having a distal end and a proximal end, the second end cap (1406) being a tapered end cap (1406) which increases in diameter from a proximal to a distal direction wherein the second end cap (1406) is positioned proximal to the tubular endoprosthesis (104), the proximal end of the second end cap (1406) further including one or more deformable raised surface features on an outside surface of the proximal end of the second end cap (1406), the second end cap (1406) being slideable along the elongate member (102); and
a covering member (106) comprising a plurality of fibers, the covering member at least partially covering and constraining the endoprosthesis (104) to a delivery diameter, and the covering member (106) extending over at least a portion of the first end cap (1402).

2. The endoprosthesis delivery system (1400) of claim 1, wherein the second end cap (1406) is positioned proximal to the endoprosthesis (104) such that the distal end of the second end cap (1406) is adjacent to a proximal end of the endoprosthesis (104).

3. The endoprosthesis delivery system (1400) of claim 2, wherein the first end cap (1402) is fixedly coupled to the elongate member (102), and wherein the second end cap (1406) is adapted to translate relative to the first end cap (1402) such that the first end cap (1402) is adapted to be drawn proximally toward the second end cap (1406) to contact the second end cap (1406).

4. The endoprosthesis delivery system (1400) of any one of claims 1 to 3, wherein the proximal end of the first end cap (1402) is deformable such that the proximal end of the first end cap (1402) can be conformed to the distal end of the endoprosthesis (104).

5. The endoprosthesis delivery system (1400) of any one of claims 1 to 4, further comprising an olive (108) situated distal to the first end cap (1402).

6. The endoprosthesis delivery system (1400) of any one of claims 1 to 5, wherein the first end cap (1402) is operable to bend along a longitudinal axis of the endoprosthesis delivery system (1400).

7. The endoprosthesis delivery system (1400) of any one of claims 1 to 6, wherein the first end cap (1402) has a compressed configuration such that the first end cap (1402) has a stored length to facilitate bending of the first end cap (1402).

8. The endoprosthesis delivery system (1400) of any one of claims 2 to 7, wherein the first end cap (1402) is tapered such that a distal end of the first end cap (1402) has a smaller diameter than the proximal end of the first end cap (1402) and wherein the second end cap (1406) is tapered such that a proximal end of the second end cap (1406) has a larger diameter than the distal end of the second end cap (1406), the proximal end of the second end cap (1406) further including one or more deformable raised surface features on an outside surface of the proximal end of the second end cap (1406).

9. The endoprosthesis delivery system (1400) of any one of claims 1 to 8, further comprising a flexible element (110) secured to the elongate member (102) and disposed over a distal edge of the endoprosthesis (104).

10. The endoprosthesis delivery system (1400) of any one of claims 1 to 9, wherein a diameter of the first end cap (1402) and the delivery diameter of the constrained endoprosthesis (104) are substantially the same.

11. The medical system of any one of claims 1 to 10, wherein the first end cap is adapted to prevent the covering member from catching on the distal end of the endoprosthesis during removal of the covering member.

12. The endoprosthesis delivery system (1400) of claim 2, wherein the second end cap (1406) comprises the one or more deformable raised surface features on its largest diameter portion.

13. The endoprosthesis delivery system (1200) of any one of claims 1 to 11, wherein the covering member (106) is configured to unravel to facilitate deployment of the constrained endoprosthesis (104).

14. The endoprosthesis delivery system (1400) of claim 1, wherein the distal end of the tubular endoprosthesis (104) is an edge located at a distal section of the elongate member (102) and the first end cap (1402) is adjacent to the edge of the tubular endoprosthesis (104).

## Patentansprüche

1. Endoprothesenabgabesystem (1400), umfassend:
ein längliches Element (102);
eine röhrenförmige Endoprothese (104), die ein distales Ende umfasst und um das längliche Element (102) angeordnet ist;
eine erste Endkappe (1402), die ein distales Ende und ein proximales Ende aufweist und so positioniert ist, dass das proximale Ende der ersten Endkappe (1402) an das distale Ende der Endoprothese (104) angrenzt;
eine zweite Endkappe (1406), die ein distales Ende und ein proximales Ende aufweist, wobei die zweite Endkappe (1406) eine sich verjüngende Endkappe (1406) ist, die dem Durchmesser nach von einer proximalen zu einer distalen Richtung zunimmt, wobei die zweite Endkappe (1406) proximal zu der röhrenförmigen Endoprothese (104) positioniert ist, wobei das proximale Ende der zweiten Endkappe (1406) ferner ein oder mehrere verformbare erhabene Oberflächenmerkmale an einer Außenoberfläche des proximalen Endes der zweiten Endkappe (1406) enthält, wobei die zweite Endkappe (1406) entlang des länglichen Elements (102) verschiebbar ist; und
ein Abdeckelement (106), das eine Vielzahl von Fasern umfasst, wobei das Abdeckelement die Endoprothese (104) mindestens teilweise abdeckt und auf einen Abgabedurchmesser beschränkt, und wobei sich das Abdeckelement (106) über mindestens einen Abschnitt der ersten Endkappe (1402) erstreckt.

2. Endoprothesenabgabesystem (1400) nach Anspruch 1, wobei die zweite Endkappe (1406) proximal zu der Endoprothese (104) positioniert ist, sodass das distale Ende der zweiten Endkappe (1406) an ein proximales Ende der Endoprothese (104) angrenzt.

3. Endoprothesenabgabesystem (1400) nach Anspruch 2, wobei die erste Endkappe (1402) fest mit dem länglichen Element (102) gekoppelt ist und wobei die zweite Endkappe (1406) dazu ausgelegt ist, sich relativ zu der ersten Endkappe (1402) umzusetzen, sodass die erste Endkappe (1402) dazu ausgelegt ist, proximal zu der zweiten Endkappe (1406) hingezogen zu werden, um die zweite Endkappe (1406) zu berühren.

4. Endoprothesenabgabesystem (1400) nach einem der Ansprüche 1 bis 3, wobei das proximale Ende der ersten Endkappe (1402) verformbar ist, sodass das proximale Ende der ersten Endkappe (1402) an das distale Ende der Endoprothese (104) angepasst werden kann.

5. Endoprothesenabgabesystem (1400) nach einem der Ansprüche 1 bis 4, ferner umfassend eine Olive (108), die distal zu der ersten Endkappe (1402) angeordnet ist.

6. Endoprothesenabgabesystem (1400) nach einem der Ansprüche 1 bis 5, wobei die erste Endkappe (1402) betätigbar ist, um sich entlang einer Längsachse des Endoprothesenabgabesystems (1400) zu biegen.

7. Endoprothesenabgabesystem (1400) nach einem der Ansprüche 1 bis 6, wobei die erste Endkappe (1402) eine komprimierte Konfiguration aufweist, sodass die erste Endkappe (1402) eine verstaute Länge aufweist, um ein Biegen der ersten Endkappe (1402) zu erleichtern.

8. Endoprothesenabgabesystem (1400) nach einem der Ansprüche 2 bis 7, wobei sich die erste Endkappe (1402) verjüngt, sodass ein distales Ende der ersten Endkappe (1402) einen kleineren Durchmesser als das proximale Ende der ersten Endkappe (1402) aufweist, und wobei die zweite Endkappe (1406) sich verjüngt, sodass ein proximales Ende der zweiten Endkappe (1406) einen größeren Durchmesser als das distale Ende der zweiten Endkappe (1406) aufweist, wobei das proximale Ende der zweiten Endkappe (1406) ferner ein oder mehrere verformbare erhabene Oberflächenmerkmale an einer Außenoberfläche des proximalen Endes der zweiten Endkappe (1406) enthält.

9. Endoprothesenabgabesystem (1400) nach einem der Ansprüche 1 bis 8, ferner umfassend ein flexibles Bauteil (110), das an dem länglichen Element (102) befestigt und über einer distalen Kante der Endoprothese (104) angeordnet ist.

10. Endoprothesenabgabesystem (1400) nach einem der Ansprüche 1 bis 9, wobei ein Durchmesser der ersten Endkappe (1402) und der Abgabedurchmesser der beschränkten Endoprothese (104) im Wesentlichen gleich sind.

11. Medizinisches System nach einem der Ansprüche 1 bis 10, wobei die erste Endkappe dazu ausgelegt ist, zu verhindern, dass sich das Abdeckelement während des Entfernens des Abdeckelements an dem distalen Ende der Endoprothese verfängt.

12. Endoprothesenabgabesystem (1400) nach Anspruch 2, wobei die zweite Endkappe (1406) das eine oder die mehreren verformbaren erhabenen Oberflächenmerkmale an ihrem Abschnitt mit dem größten Durchmesser umfasst.

13. Endoprothesenabgabesystem (1200) nach einem der Ansprüche 1 bis 11, wobei das Abdeckelement (106) dazu konfiguriert ist, sich aufzulösen, um eine Entfaltung der beschränkten Endoprothese (104) zu erleichtern.

14. Endoprothesenabgabesystem (1400) nach Anspruch 1, wobei das distale Ende der röhrenförmigen Endoprothese (104) eine Kante ist, die sich an einem distalen Bereich des länglichen Elements (102) befindet, und die erste Endkappe (1402) an die Kante der röhrenförmigen Endoprothese (104) angrenzt.

## Revendications

1. Système de pose d'endoprothèse (1400) comprenant :
un élément allongé (102) ;
une endoprothèse tubulaire (104) comprenant une extrémité distale et étant disposée autour de l'élément allongé (102) ;
un premier capuchon d'extrémité (1402) possédant une extrémité distale et une extrémité proximale, et étant positionné de sorte que l'extrémité proximale du premier capuchon d'extrémité (1402) soit adjacente à l'extrémité distale de l'endoprothèse (104) ;
un second capuchon d'extrémité (1406) possédant une extrémité distale et une extrémité proximale, le second capuchon d'extrémité (1406) étant un capuchon d'extrémité conique (1406) qui augmente de diamètre d'une direction proximale à une direction distale, ledit second capuchon d'extrémité (1406) étant positionné à proximité de l'endoprothèse tubulaire (104), ladite extrémité proximale du second capuchon d'extrémité (1406) comprenant en outre une ou plusieurs caractéristiques de surface surélevées déformables sur une surface extérieure de l'extrémité proximale du second capuchon d'extrémité (1406), ledit second capuchon d'extrémité (1406) pouvant coulisser le long de l'élément allongé (102) ; et
un élément de recouvrement (106) comprenant une pluralité de fibres, l'élément de recouvrement recouvrant au moins partiellement et contraignant l'endoprothèse (104) à un diamètre de pose, et l'élément de recouvrement (106) s'étendant sur au moins une partie du premier capuchon d'extrémité (1402).

2. Système de pose d'endoprothèse (1400) selon la revendication 1, ledit second capuchon d'extrémité (1406) étant positionné à proximité de l'endoprothèse (104) de sorte que l'extrémité distale du second capuchon d'extrémité (1406) soit adjacente à une extrémité proximale de l'endoprothèse (104).

3. Système de pose d'endoprothèse (1400) selon la revendication 2, ledit premier capuchon d'extrémité (1402) étant couplé de manière fixe à l'élément allongé (102), et ledit second capuchon d'extrémité (1406) étant adapté à se déplacer en translation par rapport au premier capuchon d'extrémité (1402) de sorte que le premier capuchon d'extrémité (1402) soit adapté à être tiré de manière proximale vers le second capuchon d'extrémité (1406) pour entrer en contact avec le second capuchon d'extrémité (1406).

4. Système de pose d'endoprothèse (1400) selon l'une quelconque des revendications 1 à 3, ladite extrémité proximale du premier capuchon d'extrémité (1402) étant déformable de sorte que l'extrémité proximale du premier capuchon d'extrémité (1402) puisse être conformée à l'extrémité distale de l'endoprothèse (104).

5. Système de pose d'endoprothèse (1400) selon l'une quelconque des revendications 1 à 4, comprenant en outre une olive (108) située de manière distale par rapport au premier capuchon d'extrémité (1402).

6. Système de pose d'endoprothèse (1400) selon l'une quelconque des revendications 1 à 5, ledit premier capuchon d'extrémité (1402) pouvant être utilisé pour se courber le long d'un axe longitudinal du système de pose d'endoprothèse (1400).

7. Système de pose d'endoprothèse (1400) selon l'une quelconque des revendications 1 à 6, ledit premier capuchon d'extrémité (1402) présentant une configuration comprimée de sorte que le premier capuchon d'extrémité (1402) présente une longueur stockée pour faciliter la flexion du premier capuchon d'extrémité (1402).

8. Système de pose d'endoprothèse (1400) selon l'une quelconque des revendications 2 à 7, ledit premier capuchon d'extrémité (1402) étant conique de sorte qu'une extrémité distale du premier capuchon d'extrémité (1402) présente un diamètre plus petit que l'extrémité proximale du premier capuchon d'extrémité (1402) et ledit second capuchon d'extrémité (1406) étant conique de sorte qu'une extrémité proximale du second capuchon d'extrémité (1406) présente un diamètre plus grand que l'extrémité distale du second capuchon d'extrémité (1406), ladite extrémité proximale du second capuchon d'extrémité (1406) comprenant en outre une ou plusieurs caractéristiques de surface surélevées déformables sur une surface extérieure de l'extrémité proximale du second capuchon d'extrémité (1406).

9. Système de pose d'endoprothèse (1400) selon l'une quelconque des revendications 1 à 8, comprenant en outre un élément flexible (110) fixé à l'élément allongé (102) et disposé sur un bord distal de l'endoprothèse (104).

10. Système de pose d'endoprothèse (1400) selon l'une quelconque des revendications 1 à 9, un diamètre du premier capuchon d'extrémité (1402) et ledit diamètre de pose de l'endoprothèse contrainte (104) étant sensiblement les mêmes.

11. Système médical selon l'une quelconque des revendications 1 à 10, ledit premier capuchon d'extrémité étant adapté à empêcher l'élément de recouvrement de s'accrocher à l'extrémité distale de l'endoprothèse pendant le retrait de l'élément de recouvrement.

12. Système de pose d'endoprothèse (1400) selon la revendication 2, ledit second capuchon d'extrémité (1406) comprenant lesdites une ou plusieurs caractéristiques de surface surélevées déformables sur sa partie de plus grand diamètre.

13. Système de pose d'endoprothèse (1200) selon l'une quelconque des revendications 1 à 11, ledit élément de recouvrement (106) étant conçu pour s'effilocher de manière à faciliter le déploiement de l'endoprothèse contrainte (104).

14. Système de pose d'endoprothèse (1400) selon la revendication 1, ladite extrémité distale de l'endoprothèse tubulaire (104) étant un bord situé au niveau d'une section distale de l'élément allongé (102) et ledit premier capuchon d'extrémité (1402) étant adjacent au bord de l'endoprothèse tubulaire (104).
